# EUROPEAN PATENT SPECIFICATION

(11) **EP 0 842 668 B1**
(45) Date of publication and mention of the grant of the patent: **04.09.2002**
(21) Application number: 96118377.9
(22) Date of filing: 15.11.1996
(51) Int. Cl.: A61K 51/12, A61K 51/10

(54) **Ex-corpore method for treating human blood cells**
Ex-Corpore Verfahren zur Behändlung von Blut-Zellen
Méthode ex-corpore pour le traitement des cellules sanguines

(43) Date of publication of application: 20.05.1998
(73) Proprietor: EUROPEAN ATOMIC ENERGY COMMUNITY (EURATOM), L-2920 Luxembourg (LU)
(72) Inventor: Koch, Lothar, 76356 Weingarten (DE); Janssens, Willem, 76694 Forst (DE); Van Geel, Jacques, 76275 Ettlingen-Oberweier (DE); Dezeure, Freddy, 3080 Tervuren (BE); Vanstraelen, Danny, 3500 Hasselt (BE)
(74) Representative: Weinmiller, Jürgen

(56) References cited:
- EP-A- 0 443 479
- EP-A- 0 585 986
- WO-A-90/15625
- WO-A-96/25520
- FR-A- 2 527 928
- DATABASE MEDLINE US NATIONAL LIBRARY OF MEDICINE (NLM), BETHESDA, MD, US AN=96279681, XP002030431 & ACTA ONCOLOGICA, vol. 35, no. 3, 1996, pages 343-355, KAIREMO K.J.: "RADIOIMMUNOTHERAPY OF SOLID CANCERS: A REVIEW."
- JOURNAL OF NUCLEAR MEDICINE, vol. 34, no. 3, 1 March 1993, pages 448-454, XP000371992 NORRGREN K ET AL: "A GENERAL, EXTRACORPOREAL IMMUNOADSORPTION METHOD TO INCREASE THE TUMOR-TO-NORMAL TISSUE RATIO IN RADIOIMMUNOIMAGING AND RADIOIMMUNOTHERAPY"
- AM. J. HEALTH-SYST. PHARM. (1996), 53(5), 521-34 CODEN: AHSPEK;ISSN: 1079-2082, 1996, XP002030533 MCCAULEY, DAYNA L.: "High-dose chemotherapy with stem-cell rescue for the treatment of breast cancer"

## Description

This invention relates to an ex-corpore method for purging human blood cells with respect to tumour cells using radioactive nuclides killing the tumour cells by use of radioactive nuclides.

Blood cells referred to in this text include both stem cells (e.g. peripheral stem cells harvests directly from the patient's blood, with or without the use of haematopoietic or colony-stimulating growth factors) and bone marrow (e.g. extracted from the patient for autologous transplant).

Purging of blood cells from tumour cells is of crucial importance for the success of high dose chemotherapy (HDCT) and total body irradiation (TBI) during autologous bone marrow transplantation (ABMT) and haematopoietic stem cell rescue (HSCR), see for example the papers:
- "New strategies in marrow purging for breast cancer patients receiving high-dose chemotherapy with autologous bone marrow transplantation" of E.J. Shpall, S.M. Stemmer, S.I. Bearman, S. Myers, M. Purdy, R.B. Jones, published in Breast Cancer Research and Treatment, 1993, 26 suppl S.19-23d
- "Autologous bone marrow transplantation vs continued chemotherapy for stage IV neuroblastoma: a nonrandomized childrens cancer group study" of D.O. Stram, K.K. Matthay, M.C. O'Leary, C.P. Reynolds, G.M. Haase, J.B. Atkinson, R.C. Seeger published in Proc Annu Meet Am Soc Clin Oncol 1995, page 14 (A1435)
- "Purging of small cell lung cancer cells from human bone marrow using ethiofs (WR-2721) and light-activated merocyanine 540 phototreatment" of R.C. Meagher, S.A. Rothman, P. Paul, P. Koberna, C. Willmer, P.A. Baucco published in Cancer Research (1989 Jul 1) 49 (13) 3637-41.

However, the removal of tumour cells and residues during purging has not yet been successfully achieved until now, see for example the papers:
- "Die Hochdosischemotherapie in der Behandlung des Mammakarzinoms: Gegenwärtiger Stand und Grenzen der Therapiemethode" of H.G. Derigs, C. Huber, M. Mahlke, K. Kolbe published in Geburtshilfe und Frauenheilkunde April 1996 56(4) 190-7
- "High-dose chemotherapy with stem-cell rescue for the treatment of breast cancer" of D.L. McCauley published in American Journal of Health-System Pharmacy Mar 1,1996 53(5) 521-34
- "Autologous bone marrow transplantation in the treatment of acute myeloid leukaemia: the Dartmouth experience and a review of literature" of L.E. Mills, G.G. Cornwell published in Cancer Investigation (1990) 8 (2) 181-90.

²¹²Bi and ²¹³Bi conjugated to monoclonal antibodies have proven to be effective in killing cancer cells in vitro and are about to be applied as in-vivo therapy (see "Cytotoxicity of ²¹³Bi and ²²⁵Ac immunoconjugates" of F.M. Kaspersen, E. Bos, A.V. Doornmalen, M.W. Geerlings, C. Apostolidis, R. Molinet published in Nuclear Medicine Communications 16, 1995). This would involve the injection of radioactivity in a patient where it could lead to damage of healthy cells located in organs which have some absorption properties for the monoclonal antibodies and their conjugates.

The document UK-A-2 122 641 discloses a method for manufacturing and purifying radiolabelled metal chelate conjugated monoclonal antibodies in view of a possible use in in-vivo cancer therapy. The document ACTA ONCOLOGICA, 1996, 35(3), 343-55 discloses a method for reducing the myelotoxicity by an extracorporeal immunoadsorption of radiolabelled antibodies.

The invention as defined in the annexed claims proposes a new method of purging blood which excludes or largely reduces injection of radiolabelled compounds into the patient.

This invention can be applied in Autologous Bone Marrow Transplantation (ABMT) and/or Haematopoietic Cell Rescue (HSCR) performed in the frame of High-Dose Chemotherapy (HDCT) and/or Total Body Irradiation (TBI). In the described application, killing the tumour cells contaminating the bone marrow and the stem cells takes place ex-corpore. The tumour cells are destroyed by short-lived radionuclides, which spare largely other cells due to the short decay range and the selectivity of the carriers for the tumour cells.

During HDCT and/or TBI the blood cells for the autologous transplant are cryopreserved for several days. During this period they can be purged at external facilities equipped to handle such radioactive nuclides, eliminating the need of hospitals to install and maintain expensive specialised laboratories. This is contrary to the in-vivo treatment, which requires an on-site preparation of the radioimmunoconjugate loaded with the alpha-emitter (e.g. ²¹³Bi) prior to injection because of the short half life and the instability of the conjugate.

The required activity for the purging method, as described in this invention, is expected to be considerably smaller than in the case of the in-vivo treatment. The main reason is that only the bone marrow and/or stem cells are treated to kill the cancer cells. The major amount of cancer cells in the patient will indeed be killed in the associated HDCT and/or TBI.

If the half lives of the radionuclides used for the purging are short enough, the transplant will be practically free of artificial radioactivity and does not expose the patient in excess of the maximum permissible annual limit for intake of these nuclides (e.g. Richtlinie 96/29/Euratom des Rates vom 13. Mai 1996, referred to in Amtsblatt der Europäischen Gemeinschaften, L159, 29 June 1996).

The possible long term effects of the residues of the alpha-emitters in the patient's body (mainly heavy metals) are not yet known. It is clear however than any associated risk will be strongly reduced when the alpha-activity is only used for the purging (as described in this invention) compared to the in-vivo treatment.

A dose of less than 100 mCi of ²¹²Bi/²¹³Bi is estimated to be sufficient for purging. This activity will decay to the maximum permissible annual uptake of alpha-emitting Binuclides of 10⁻⁶Ci within one day regardless of any purification of the transplant after purging. This avoids the patient to be exposed beyond limits for artificial radioactivity.

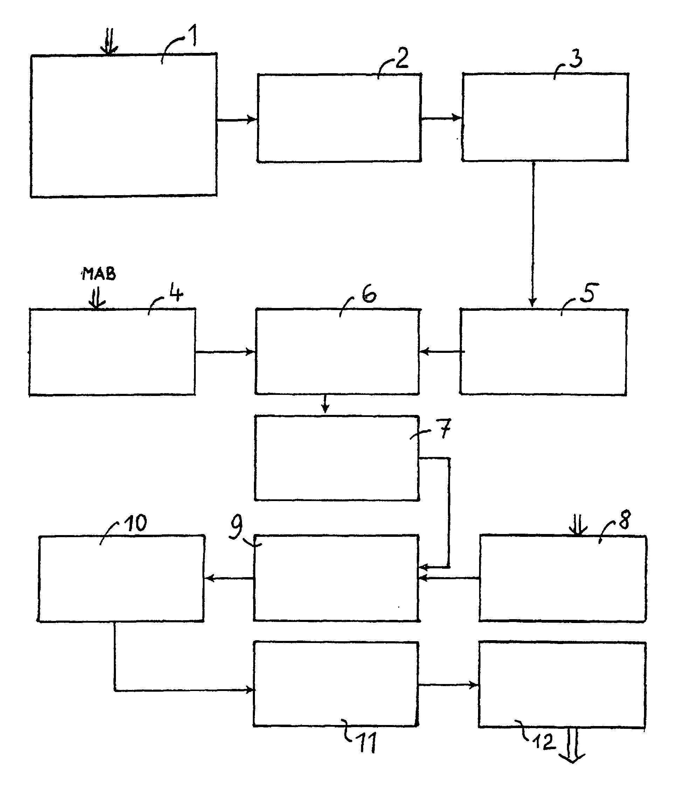

A central laboratory is provided to use the products and implement the techniques described here. The structure of this central laboratory depends on the radionuclides used. Two examples are given here. When using pure ²¹³Bi (e.g. separated from irradiated ²²⁶Ra targets or ²³³U decay), the central laboratory is schematically presented in the enclosed unique figure of the drawings. It consists of three installations, grouped under one roof. If a mixture of ²¹²Bi and ²¹³Bi were used (e.g. separated after neutron irradiation of a ²²⁶Ra target - see patent EP 0 443 479 B1) the set-up of the central laboratory would be slightly different. The main differences are the source materials for the radionuclides and the use of shielded facilities in the case of the ²¹²Bi/²¹³Bi mixture. The operations to be performed in the sterile installations are similar in both cases.

The installation shown in the drawings includes in a first part all the equipment, tools and materials to produce the required radionuclides upon request:
- a hot cell 1 to separate the ²¹³Bi parent nuclides from the source material,
- a glovebox 2 to fabricate the ²¹³Bi generator,
- a set of analysis tools 3 (e.g. α and γ spectrometers) to determine the radiochemical yield and purity of the source materials and daughter products.

In the case where ²²⁹Th is used as mother nuclide, the following actions are performed:

²²⁹Th is absorbed from 8 M HNO₃ on a Dowex 1x8, 100-200 mesh resin batch (for 63 mg Th, about 1 ml resin is taken). The breakthrough of about 2 percent Th is removed by passing through a second 1.5x20 cm column of the same resin. The effluent contains ²²⁵Ra, ²²⁵Ac and decay-products (i.e. most ²²⁵Ac). Eventual Th traces still present in the effluent are absorbed on a third column of Dowex 50 Wx8, 100-200 mesh resin column (0.5x30cm).

The chemical purity of the reagents for this production is known and checked by Inductively Coupled Plasma Mass Spectrometry.

The radiochemical purity, already at this stage, is determined using alpha- and gamma-spectrometry.

According to a variant, a second source of ²²⁵Ac is the direct production by irradiation of ²²⁶Ra in a cyclotron. A chemical separation step in this case is required to separate the Ra from the Ac. This is done in the following way:
- Boiling the Ac-Ra-mixture in 8 M HNO₃,
- Evaporating the Ac³⁺ and Ra²⁺ in the solution and redissolving in 2 M HCl
- Fixing this product onto a previously conditioned column filled with Dowex 50Wx8 (100-200 mesh SERVA)
- Passage of 1 bed volume 2 M HCl eliminates ²²¹Fr, ^{212,213}Bi, ²⁰⁹Pb
- Passage of 10 bed volumes 2 M HNO₃ eliminates ²²⁶Ra
- Elution of the ²²⁵Ac product is then performed by passing 5 bed volumes 4 M HNO₃.

²¹³Bi can be obtained from ²²⁵Ac according to the following method:
- The Ac, which is fixed typically on a cation exchanger, can be "milked" to extract ²¹³Bi with both a good yield and high purity.
- A glass column (50*5 mm) with 0.4 g Dowex 50 Wx8 (100-200 mesh SERVA) is used which is conditioned by successive passages of 5 bed volumes 9 M HCl, 5xH₂O and 5x2 M HCl.
- The ²²⁵Ac product is evaporated, redissolved in 0.2 ml HCl and fixed on to the column.
- The accumulation of purified ²²⁵Ac is followed by a quality control procedure consisting of alpha-spectrometry, gamma-spectrometry and/or Inductively Coupled Plasma Mass Spectrometry.

Thereafter the produced short lived alpha-emitting radionuclides must be coupled to the monoclonal antibodies (MAB) or any other appropriate carriers e.g. hormones and proteins specific to the respective tumour cells. The MAB and other carriers are conjugated to the appropriately treated and conditioned radionuclides. The radioimmunoconjugate undergoes extensive quality control before being transferred to the next installation. This part of the laboratory therefore possesses:
- a sterile bank 4 to prepare the MAB or other carriers after receipt from outside
- a glovebox 5 to produce the short lived radionuclides (here ²¹³Bi)
- a glovebox 6 to conjugate the ²¹³Bi to the MAB or other carriers
- A quality control station 7 to check e.g. the stability and loading efficiency of the radioimmunoconjugate.

The following actions are performed:
- ²¹³Bi is eluted from the above described generator in 2 bed volumes 0.1 M NaI/HCl.
- This elution procedure is repeated until the ²²⁵Ac activity is exhausted.

To the monoclonal antibodies or other carriers chemical ligands are conjugated (e.g. chelates) in order to load the radionuclide. Alternatively, the chemically complexed radionuclide is condensed to -SH, -NH oder -OH groups of the monoclonal antibodies or other carriers.

The conjugate has to be purified from not reacted material and sterilised.

Monoclonal antibodies and other carriers for different cancer types are commercially available and some have been used for therapeutic purposes. They will be selected according to the cancer type for the subsequent purging process taking into account:
- High selectivity of the antibodies to the tumour cell type
- Stability of the conjugate complex in retaining ²¹³Bi

The method is described for conjugation of BB4 IgG with Benzyl DPTA complex and ²¹³Bi.
- ²¹³Bi is brought in solution in a colloid-free form and adjusted to pH 5 by the addition of 50 microliter of 10 M NaOH and 200 microliter of a citrate/acetate buffer 0.25/1.3 M, pH 5.
- The immunoconjugate is brought to the appropriate conditions to be mixed with the radioisotope solution described above (a different approach can be followed in first complexing the radioisotope with an organic compound which is condensed to the unchanged monoclonal antibody or other carrier).
- This mixture of the radioisotope solution and the immunoconjugate can be incubated
- The quality of the loading of ²¹³Bi to the immunoconjugate is being checked by TLC.
- To remove the non-bound ²¹³Bi of bound ²¹³Bi to the radioimmunoconjugate, a filtration/separation step is provided, using a working principle similar to that of a molecular sieve such as Sephadex G-100 or PD-10 exclusion-diffusion gel, or similar.
- Analysis will check for radiochemical impurities by gamma-spectrometry and alpha-spectrometry.

Thereafter, the actual purging of the blood cells is performed. After stem cell harvest or bone marrow extraction the blood cells arrive from the hospitals and will be prepared for the mixing with the conjugated nuclide. After this mixing, the purging of the blood stem cells/bone marrow takes place, the crucial aspect being that this treatment takes place outside of the patient's body. The treated transplant will then be subject to a quality control before being prepared to be shipped back to the hospital (in cryopreserved form). For this purpose the following equipment, techniques and materials are required in this installation:
- a receipt equipment 8 for the blood cells
- a mixing equipment 9 for the blood cells and the conjugated radionuclide
- a purging equipment 10 (e.g. incubators - see below) for the transplant after the treatment
- a quality control station 11 to check the purification of transplant
- an equipment 12 for cryopreservation and for preparing the transplant for transport before sending it back to the hospital.

The following actions are performed:

Prior to the purging of the blood cells (bone marrow and stem cells) the cytotoxicity of the ²¹³Bi immunoconjugate for the tumour cells is tested as follows:

For this purpose monolayers of the tumour cells are brought in contact with the radioimmunoconjugate.

The survival rate of the cells, brought in contact with the radioimmunoconjugate, is tested:

The MTT check, based on chemical interaction with the cells inducing colour changes, uses an absorption spectrometer to determine the survival rate.

The number of radionuclide atoms needed depends on the tumour size. Accordingly the selectivity of the immunoconjugate to the tumour cell in question has to be determined in advance.

From the foregoing description and the annexed claims it will be clear that the present invention differs from a medical treatment of human patients by the fact that the method is applied ex-corpore in a radiological laboratory on extracted blood stem cells and is not practised on the human body itself. This particularity offers decisive advantages with respect to the in-vivo treatment:

The amount of activity required (only to kill residual tumour cells in the bone marrow or stem cell transplant) is minimised and the risk to damage other tissues is largely eliminated contrary to in-vivo treatment.

The purged blood cells are returned to the patient body without radioactivity (i.e. below the maximum permissible annual limit for intake, see e.g. "Richtlinie 96/29/Euratom des Rates vom 13. Mai 1996" referred to in Amtsblatt der Europäischen Gemeinschaften, L159, 29 June 1996) due to the use of short-lived radionuclides and the treatment ex-corpore.

The treatment is performed outside the hospital in a central laboratory having the required operating licenses and installation "under one roof" and being able to serve more than one hospital.

## Claims

1. An ex-corpore method of treating human blood cells incorporating tumour cells, consisting of stem cells extracted from said blood and/or bone marrow extracted from the patient for autologous transplant, in particular in the frame of High Dose Chemotherapy and/or Total Body Irradiation, **characterized by** the following steps:
a) carriers selective for specific tumour cells are conjugated to radionuclides which emit short range alpha particles with high linear energy transfer, forming a radioimmunoconjugate,
b) a filtration/separation step is performed on the radioimmunoconjugate ensuring the removal of non-conjugated radionuclides,
c) stem cells extracted from said blood and/or bone marrow extracted from a patient for autologous transplant are mixed ex-corpore with said conjugated radionuclides,
d) after an incubation period a purging of the blood stem cells and/or bone marrow is performed.

2. A method according to claim 1, **characterized in that** the radionuclides are selected from ²²⁵Ac, ²¹²Bi, ²¹³Bi and mixtures thereof.

3. A method according to any one of the preceding claims, **characterized in that** chemical ligands, e.g. chelates, are conjugated to the carriers in order to load the radionuclides..

4. A method according to any one of claims 1 to 2, **characterized in that** the chemically complexed radionuclide is condensed to -SH, -NH or -OH groups of the carrier.

5. A method according to any one of claims 1 to 4, **characterized in that** the carriers are selected from monoclonal antibodies, hormones and proteins specific to the respective tumour cells.

6. A method according to any one of claims 1 to 5, **characterized in that** the conjugated radionuclides are sterilised prior to step c).

## Patentansprüche

1. Verfahren zur ex-corpore-Behandlung von menschlichen Blutzellen, die Krebszellen enthalten, bestehend aus Stammzellen, die aus dem Blut extrahiert wurden, und/oder Rückenmark, das aus dem Patienten für eine Eigentransplantation entnommen wurde, insbesondere im Rahmen einer Hochdosis-Chemotherapie und/oder Ganzkörperbestrahlung, **gekennzeichnet durch** die folgenden Schritte:
a) Für spezifische Krebszellen spezifische Träger werden an Radionuklide gebunden, die Alphapartikel kurzer Reichweite mit hoher linearer Energietransferrate emittieren, sodaß sich ein Radio-Immunokonjugat ergibt;
b) das Radio-Immunokonjugat wird gefiltert, wodurch nicht konjugierte Radionuklide entfernt werden;
c) Stammzellen, die aus dem Blut extrahiert wurden, und/oder Rückenmark, das dem Patienten für eine Eigentransplantation entnommen wurden, werden ex-corpore mit den konjugierten Radionukliden gemischt;
d) nach einer Inkubationsperiode werden die Blutstammzellen und/oder das Rückenmark gereinigt.

2. Verfahren nach Anspruch 1, **dadurch gekennzeichnet, daß** die Radionuklide ausgewählt werden aus ²²⁵AC, ²¹²Bi, ²¹³Bi und deren Gemischen.

3. Verfahren nach einem beliebigen der vorstehenden Ansprüche, **dadurch gekennzeichnet, daß** chemische Bindemittel, z.B. Chelate, mit den Trägern konjugiert werden, um die Radionuklide zu binden.

4. Verfahren nach einem beliebigen der Ansprüche 1 und 2, **dadurch gekennzeichnet, daß** das chemisch komplexierte Radionuklid an -SH, -NH oder -OH-Gruppen des Trägers kondensiert wird.

5. Verfahren nach einem beliebigen der Ansprüche 1 bis 4, **dadurch gekennzeichnet, daß** die Träger aus monoklonalen Antikörpern, Hormonen und Proteinen ausgewählt werden, die für die jeweiligen Krebszellen spezifisch sind.

6. Verfahren nach einem beliebigen der Ansprüche 1 bis 5, **dadurch gekennzeichnet, daß** die konjugierten Radionuklide vor dem Schritt c) sterilisiert werden.

## Revendications

1. Procédé ex-corpore de traitement de cellules sanguines humain incorporant des cellules tumorales, constituées de cellules souches extraites dudit sang et/ou de moelle osseuse extraite du patient pour une transplantation autologue, en particulier dans le cadre d'une chimiothérapie à haute dose et d'une irradiation totale du corps, **caractérisé par** les étapes suivantes:
a) des porteurs sélectifs pour des cellules tumorales spécifiques sont conjugués à des radionucléides qui émettent des particules alpha à courte portée avec un transfert élevé d'énergie linéaire, formant un radioimmunoconjugué,
b) une étape de filtration/séparation est réalisée sur le radioimmunoconjugué, assurant l'élimination de radionucléides non conjugués,
c) des cellules souches extraites dudit sang et/ou de la moelle osseuse extraite d'un patient pour une transplantation autologue sont mélangées ex corpore avec lesdits radionucléides conjugués,
d) après une période d'incubation, une purge des cellules souche et/ou de la moelle osseuse est effectuée.

2. Procédé selon la revendication 1, **caractérisé en ce que** les radionucléides sont choisis de 225AC, 212Bi, 213Bi et leurs mélanges.

3. Procédé selon l'une quelconque des revendications précédentes, **caractérisé en ce que** des ligands chimiques, i.e. des chélates, sont conjugués aux porteurs pour charger les radionucléides.

4. Procédé selon l'une quelconque des revendications 1 et 2, **caractérisé en ce que** le radionucléide complexé chimiquement est condensé à des groupes -SH, -NH or -OH du porteur.

5. Procédé selon l'une quelconque des revendications 1 à 4, **caractérisé en ce que** les porteurs sont choisis des anticorps monoclonaux, des hormones et protéines spécifiques aux cellules tumorales respectives.

6. Procédé selon l'une quelconque des revendications 1 à 5, **caractérisé en ce que** les radionucléides conjugués sont stérilisés avant l'étape c).
